(19) Europäisches Patentamt
European Patent Office
Office européen des brevets

(11) Publication number: **0 067 666**
**B1**

(12) **EUROPEAN PATENT SPECIFICATION**

(45) Date of publication of patent specification: **30.10.85**

(21) Application number: **82302997.0**

(22) Date of filing: **10.06.82**

(51) Int. Cl.$^4$: **C 07 D 215/56,** C 07 D 471/04, C 07 C 53/126, C 07 C 101/22, A 61 K 31/495

(54) **Salts of antimicrobial naphthyridine and quinoline compounds and their production.**

(30) Priority: **11.06.81 US 272601**
**24.12.81 US 334182**

(43) Date of publication of application:
**22.12.82 Bulletin 82/51**

(45) Publication of the grant of the patent:
**30.10.85 Bulletin 85/44**

(84) Designated Contracting States:
**AT BE CH DE FR GB IT LI LU NL SE**

(56) References cited:
**EP-A-0 009 425**
**EP-A-0 035 862**

(73) Proprietor: **WARNER-LAMBERT COMPANY**
**201 Tabor Road**
**Morris Plains New Jersey 07950 (US)**

(72) Inventor: **Mich, Thomas Frederick**
**915 Sunset Road**
**Ann Arbor Michigan 48103 (US)**

(74) Representative: **Jones, Michael Raymond et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 067 666 B1

**Description**

This invention relates to salts of antimicrobial naphthyridine and quinoline compounds, and their production.

In general, medications are most conveniently administered to humans by the oral route. Thus, stable oral dosage forms such as tablets, capsules and oral liquids are most usually produced. In such oral dosage forms, the solubility of the contained active component is generally not a problem since absorption is facilitated by the prolonged length of time the material spends in the alimentary canal, and by the general digestive processes. It is likely, however, that highly insoluble materials will be only slowly absorbed upon oral administration and that a portion of the dosage may be excreted, thereby not providing the full desired medical effect. Methods of improving the solubility of such materials must therefore be applied in order to produce an efficacious oral dosage form. One such method known in the art is the preparation of a soluble salt of the medicament.

In many instances, the oral route of administration is not usable or may not be preferred. For example when treating microbial infections in humans a parenteral route of administration is often preferred or may in fact be necessary.

Thus in order to rapidly obtain a high blood level of a particular antimicrobial agent, intravenous administration is often employed. Slow intravenous infusion may also be employed to maintain a particular blood level over a prolonged period of time. Intramuscular and intravenous injections may also be employed when treating a pediatric, infirm or unconscious patient.

The characteristics of the active component necessary for preparing a stable parenteral dosage form or for preparing a stable dosage form which may be constituted into a parenteral dosage form shortly before use are different from the requirements for an oral dosage form which contains the same active ingredient. Thus, for parenteral formulations the active component must itself possess or be convertible to a derivative (e.g. salt) which has at least the following characteristics:

a) the derivative must have high water solubility at a pH which is compatible with blood and muscular tissue and which is non-injurious to cell structure, preferably from pH 4 to 8;

b) the derivative must have stability in solution, preferably at room temperature and in the presence of air;

c) the agent used to create the derivative (i.e. an acid or base) must be relatively non-toxic and acceptable to the Drug Regulatory Agencies;

d) the same agent should be relatively inexpensive;

e) the derivative should be readily formed and lyophilizable to a stable form; and

f) the derivative should be compatible with and sufficiently soluble in standard parenteral solutions for convenient administration.

Recently, two new antimicrobial agents have been reported. Thus, the antimicrobial properties of 1-ethyl-6-fluoro-1,4-dihydro-4-oxo-7-(1-piperazinyl)-3-quinolinecarboxylic acid (hereinafter referred to as compound A) are reported in Antimicrobial Agents and Chemotherapy *17* (1980) 103—108, and in Antimicrobial Agents and Chemotherapy *19* (1981) 188—189. The antimicrobial properties of 1-ethyl-6-fluoro-1,4-dihydro-4-oxo-7-(1-piperazinyl)-1,8-naphthyridine-3-carboxylic acid (hereinafter referred to as compound B) are reported in Current Chemotherapy and Infectious Disease, Proceedings of the 11th International Congress of Chemotherapy and the 19th Interscience Conference of Antimicrobial Agents and Chemotherapy Vol. 1 Amer. Soc. for Microbiology 1980, Washington, D.C. page 451.

Compound A is also described in United States Patent No. 4,146,719. However, compound A is amphoteric and does not possess a high degree of water solubility, and that United States patent discloses the preparation of only the hydrochloride salt. For reasons discussed hereinafter, this salt and many other salts of compound A prepared from a variety of acids and bases are unsuitable for preparing a parenteral dosage form containing compound A. It would therefore be beneficial and advantageous to provide derivatives of compound A which possess the desired characteristics described above in order that a useful parenteral dosage form can be prepared.

Compound B as the hydrochloride is prepared in Example 1 of published British Patent Application No. 2,034,698. No dosage forms are described.

Compound B is also described in European Patent Application Publication No. 9425. Compound B is amphoteric and does not possess a high degree of water solubility. The patent discloses *inter alia* that salts of compound B may be formed from "various inorganic and organic acids, and examples of suitable acids are hydrochloric acid, acetic acid, lactic acid, succinic acid, lactobionic acid and methanesulphonic acid. Especially preferred salts are the hydrochlorides or methanesulphonates." The European Patent Application Publication No. 9425 also mentions that oxalic acid may be used to prepare a salt. For reasons discussed hereinafter, these salts as well as many other salts prepared from a variety of acids and bases are unsuitable for preparing a useful parenteral dosage form containing compound B.

In describing the use of compound B, European Patent Application Publication No. 9425 states as follows:

"The compounds of this invention may be used as medicines, for example, in the form of pharmaceutical preparations containing them in admixture with an organic or inorganic pharmaceutically acceptable solid or liquid adjuvants *suitable for oral or topical administration."* (Emphasis added).

The patent goes on to state that "The pharmaceutical preparations may be powder, granules, tablets, ointments, suppositories, creams, capsules, etc."

Further, all *in vivo* test procedures reported in the published patent application, i.e. Examples B to G, utilize the oral route of administration. The only examples of dosage form preparation, Examples H and J, are for oral dosage forms; namely capsules and tablets respectively.

It is not unreasonable to assume that the inventors responsible for the invention which is the subject of that published European patent application recognized that a useful parenteral dosage form could not be readily prepared from compound B, since the preparation and use of these important dosage forms is not taught or contemplated by that published application.

It would therefore be beneficial and advantageous to provide derivatives of compound B which possess the desired characteristics described above in order that a useful parenteral dosage form can be prepared.

One aspect of the present invention provides a compound having the following general formula C:

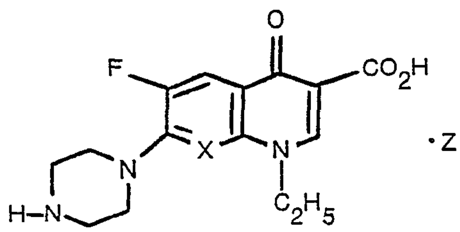

(C)

wherein X is N or CH and Z is galacturonic, aspartic, gluconic or glutamic acid.

One sub-class of compounds of the present invention are those having the following general formula D:

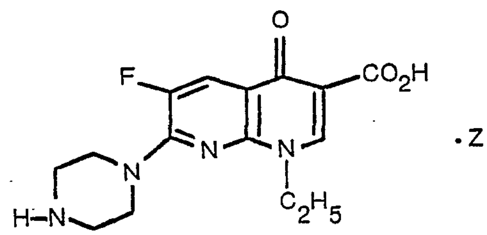

(D)

wherein Z is galacturonic, aspartic, gluconic or glutamic acid.

Another sub-class of compounds of the present invention are those having the following general formula E:

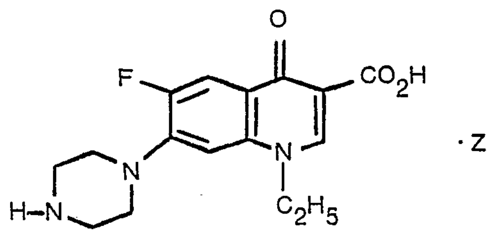

(E)

wherein Z is galacturonic, aspartic, gluconic or glutamic acid.

The present invention thus embraces, individually, the following specific compounds:—

1-ethyl-6-fluoro-1,4-dihydro-4-oxo-7-(1-piperazinyl)-1,8-naphthyridine-3-carboxylic acid galacturonate;
1-ethyl-6-fluoro-1,4-dihydro-4-oxo-7-(1-piperazinyl)-1,8-naphthyridine-3-carboxylic acid aspartate;
1-ethyl-6-fluoro-1,4-dihydro-4-oxo-7-(1-piperazinyl)-1,8-naphthyridine-3-carboxylic acid gluconate;
1-ethyl-6-fluoro-1,4-dihydro-4-oxo-7-(1-piperazinyl)-1,8-naphthyridine-3-carboxylic acid glutamate;
1-ethyl-6-fluoro-1,4-dihydro-4-oxo-7-(1-piperazinyl)-3-quinolinecarboxylic acid galacturonate;
1-ethyl-6-fluoro-1,4-dihydro-4-oxo-7-(1-piperazinyl)-3-quinolinecarboxylic acid aspartate;
1-ethyl-6-fluoro-1,4-dihydro-4-oxo-7-(1-piperazinyl)-3-quinolinecarboxylic acid gluconate; and
1-ethyl-6-fluoro-1,4-dihydro-4-oxo-7-(1-piperazinyl)-3-quinolinecarboxylic acid glutamate.

The present invention also provides as a further aspect as antibacterial pharmaceutical composition comprising a chemical compound having the following general formula C:

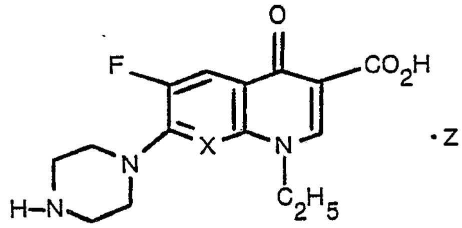

(C)

wherein X is N or CH and Z is galacturonoic, aspartic, gluconic or glutamic acid; in combination with a pharmaceutically acceptable carrier.

One sub-class of pharmaceutical compositions according to the present invention comprises a chemical compound having the following general formula D:

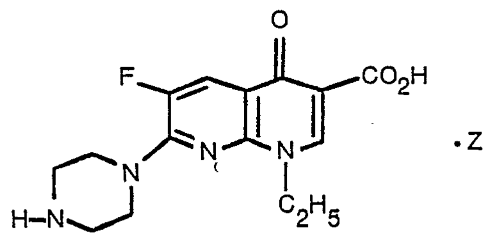

(D)

wherein Z is galacturonic, aspartic, gluconic or glutamic acid; in combination with a pharmaceutically acceptable carrier.

A further sub-class of pharmaceutical compositions according to the present invention comprises a chemical compound having the following general formula E:

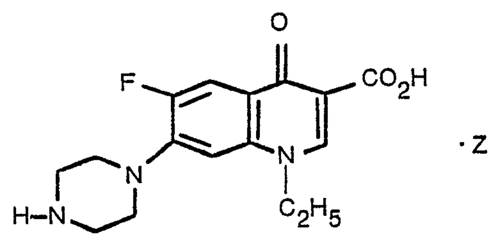

(E)

wherein Z is galacturonic, aspartic, gluconic or glutamic acid; in combination with a pharmaceutically acceptable carrier.

Preferably the pharmaceutical compositions of the present invention are parenteral antibacterial pharmaceutical compositions.

The compounds of the present invention may be used in the treatment of a mammal suffering from a bacterial infection by administering an effective amount of a chemical compound having the following general formula C, or a pharmaceutical composition including that compound, to the mammal:

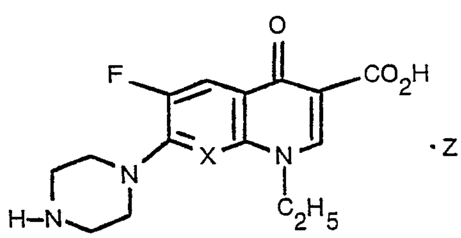

(C)

wherein X is N or CH, and Z is galacturonic, aspartic, gluconic or glutamic acid.

Preferably the treatment of the mammal involves parenterally treating the mammal suffering from a bacterial infection, by parenteral administration of an effective amount of the pharmaceutical composition of the present invention to the mammal.

A further aspect of the present invention provides a process for producing a compound having the following general formula C:

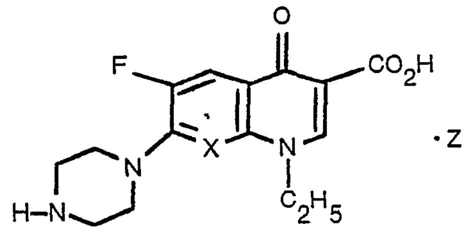

(C)

wherein X is N or CH and Z is galacturonic, aspartic, gluconic or glutamic acid; which process comprises reacting a compound having the following general formula:

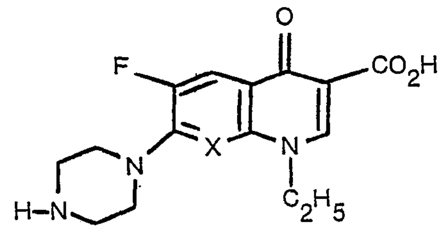

wherein X is defined above, with an acid of formula Z as defined above.

Compound A, 1-ethyl-6-fluoro-1,4-dihydro-4-oxo-7-(1-piperazinyl)-3-quinolinecarboxylic acid, may be prepared as described in United States Patent No. 4,146,719 or by variations of the procedures described therein.

Compound B, 1-ethyl-6-fluoro-1,4-dihydro-4-oxo-7-(1-piperazinyl)-1,8-naphthyridine-3-carboxylic acid, may be prepared as described in European Patent Application Publication No. 9425 or by variations of the procedures described therein.

Compounds A and B may be converted to their corresponding acid addition salts by treatment with organic and inorganic acids in known manner. Thus compounds A and B may be treated in solution or in suspension with a solution or suspension of the desired acid in the same or in a different solvent. The choice of the proper solvent or solvent mixture is within the skill of those familiar with the art; the preferred solvent of the invention is water. When preparing a salt in water, a suspension of compound A or B in water is stirred with the chosen acid which is either soluble in water or also in suspension. The mixture may be warmed briefly to aid solubility and salt formation. For example the suspension may be warmed to, say, 60°C and then allowed to return to room temperature with stirring. When preparing a gluconate salt, it has been found to be particularly convenient to prepare the gluconic acid *in situ* from gluconic acid delta lactone.

Thus, the gluconic acid delta lactone is added to a water suspension of compound A or B and is stirred for about 20 hours at room temperature. Briefly heating this mixture to about 60°C will reduce the amount of time necessary for complete salt formation to about three hours. Modification of this procedure to optimize salt formation is within the competence of those skilled in the art.

The compound A or B may be mixed with the salt-forming reagent in approximately equimolar quantities, or if desired an excess of the salt-forming reagent may be utilized. The resulting salt may be recovered by standard methods, for example lyophilization. The salt may then be purified by recrystallization if desired.

The terms "galacturonic acid", "galacturonate", "aspartic acid", "asparate", "glutamic acid", "glutamate", "gluconic acid", "gluconate" and "gluconic acid delta lactone" when utilized herein are intended to include the individual isomers of these reagents as well as mixtures of their respective isomers. Thus, the invention contemplates the use of the *D*, *L* and *DL* mixtures of the salt-forming reagents, for example. The preferred forms are the naturally occurring forms (*L* for the amino acids and *D* for gluconic acid, gluconic acid delta lactone and galacturonic acid).

The compounds of the present invention can exist in unsolvated as well as solvated forms, including hydrated forms. In general, the solvated forms, with pharmaceutically acceptable solvents such as water and ethanol, are equivalent to the unsolvated forms for the purposes of the present invention. The salts of the present invention are the galacturonate, the aspartate, the glutamate and the gluconate salts of compounds A and B. The gluconate is the preferred salt and is especially preferred when prepared from *D*-gluconic acid which is generated *in situ* from *D*-gluconic acid delta lactone.

Upon investigation of many pharmaceutically acceptable acid addition and base addition salts that were prepared from compounds A and B, it was observed tha the galacturonate, aspartate, glutamate and gluconate salts, and in particular the gluconate salts, possess the characteristics necessary for preparing useful parenteral dosage forms containing compounds A and B. The usefulness of these particular salts for

the purpose of preparing parenteral dosage forms from compounds A and B is surprising and could not have been predicted from the prior art. Thus, one skilled in the art would have assumed that useful parenteral dosage forms containing compounds A and B could have been prepared from the acid addition salts disclosed in the prior art references. Surprisingly this was not the case, and it is particularly surprising that, in the present instance, the use of the common hydrochloride salt is precluded for preparing parenteral dosage forms for *both* compounds A and B, whereas suitable parenteral dosage forms can be obtained by the use of the galacturonate, aspartate, glutamate or gluconate salts.

The following Table 1 is a compilation of solubility and pH data which demonstrates that the majority of pharmaceutically acceptable salts prepared from compounds A and B cannot be utilized to prepare a useful parenteral dosage form containing these compounds.

TABLE I
Solubility Screening Data*

| Acid, Amino Acid or Base | Conc. of CPD A mg/ml | pH of Solution | Conc. of CPD B mg/ml | pH of Solution |
|---|---|---|---|---|
| Citric | 1.5 | 3.32 | 1.3 | 3.14 |
| Tartaric | 3.5 | 3.02 | 7.7 | 3.46 |
| Oxalic | 19.8 | 2.14 | 2.8 | 1.76[b] |
| Maleic | 45.1 | 2.79 | 7.75 | 2.60 |
| Lactobionic | 46.5 | 4.65 | 99 | 3.6[b] |
| Succinic | 128 | 4.25 | 75 | 4.45[b] |
| Gluconic[d] | 182 | 4.3 | 168 | 4.6 |
| Hydrochloric | 201 | 1.52[a] | 7 | 1.83[c] |
| Acetic | 235 | 5.3 | 195 | 4.97[c] |
| Galacturonic | 166 | 5.44 | 155 | 4.78 |
| Methane Sulphonic | 175 | 1.33 | 211 | 1.67[c] |
| Isethionic | 175 | 1.35 | 220 | 1.01 |
| Lactic | 198 | 4.05 | 217 | 4.24[b] |
| Glycine | 0.9 | 6.14 | 1.2 | 6.87 |
| D,L-Alanine | 0.9 | 6.44 | 3.4 | 7.76 |
| L-Phenylalanine | 1.33 | 6.3 | 1.5 | 6.8 |
| L-Leucine | 0.9 | 6.2 | 1.1 | 6.9 |
| L-Histidine | 0.7 | 7.4 | 1.1 | 7.53 |
| L-Tryptophan | 0.5 | 6.65 | 2.0 | 7.08 |
| L-Proline | 0.9 | 6.28 | 1.1 | 6.92 |
| L-Serine | 1.0 | 6.24 | 1.3 | 6.70 |
| L-Lysine | 4.2 | 9.5 | 4.3 | 9.4 |
| L-Arginine | 11.1 | 9.8 | 6.7 | 9.7 |
| L-Aspartic | 174 | 4.88 | 165 | 4.64 |
| L-Glutamic | 174 | 5.08 | 160 | 4.98 |
| L-Cysteic | 166 | 2.92 | 221 | 2.78 |
| Diethanolamine | 7.5 | 9.68 | 4.3 | 9.7 |
| Choline | 53.5 | 10.34 | 38.2 | 9.8 |
| Sodium Hydroxide | 131 | 10.0 | 75 | 10.5[b] |

Footnotes to Table I.

[a] Salt prepared in United States Patent No. 4,146,719.

[b] Salt disclosed in European Patent Application No. 9425.

[c] Salt prepared in European Patent Application No. 9425.

[d] Commercially available gluconic acid solution utilized to prepare salt.

The data collected in Table I was obtained by the following procedure.

* SOLUBILITY SCREENING PROCEDURE

The amount of Compound A or Compound B calculated for selected concentrations was weighed into volumetric flasks. Equimolar quantities of combining acid, amino acid, or base were added either as solutions or in dry form. Deionized water was added to bring the volume to that desired for the preselected concentrations. All flasks were sonicated for 15 minutes, allowed to stand at room temperature for 30 minutes, then filtered through a fine frit sintered glass funnel. After the pH of the filtrate was taken, suitable dilutions were made into 0.1 N HCl for ultraviolet spectrophotometric assay of concentration. Ten fold dilutions of the original filtrates were made into standard I.V. solutions. These dilutions were observed for appearance of precipitate. (The results of the dilution experiments are reported in Tables III and IV *infra*.)

In the solubility screen, the initial experiment was performed in order to prepare solutions which would contain a final concentration of about 25 mg/ml of either compound A or B. Those acids or bases which did not solubilize sufficient material to provide this concentration were not considered useful. The acids or bases which were judged were then utilized to produce solutions containing at least 150 mg/ml of compound A or B. These more concentrated solutions which in addition had a pH which was in the range from 4 to 8 were considered to have successfully passed this screen procedure.

The unpredictable characteristics of the salts prepared from Compounds A and B are clear from consideration of the results reported in Table I. These characteristics were considered in view of the necessary requirements (a—f) outlined above for preparing a useful parenteral dosage form. Thus, compound A hydrochloride possesses adequate solubility but an unusable pH, while compound B hydrochloride has not only inadequate solubility but an unusable pH as well. The methane sulphonates and isethionates of both compounds A and B possess good solubility but are unsatisfactory on the basis of pH; likewise, both salts prepared from L-cysteic acid. The acetate salts of compounds A and B both appear to be useful, but they cannot be successfully lyophilized and therefore are of limited utility. From the data in Table 1, the lactate salts appear to be useful; however, lactic acid cannot be consistently obtained in a satisfactory grade in presently available commerical forms and is therefore not useful. The choline and sodium salts were ruled out based on the pH of their respective solutions.

Thus, the useful salts were found to be the galacturonate, the aspartate, the glutamate and the gluconate.

Table II further confirms the results in Table 1; but focuses on the use of selected lyophilized and crystalline salts.

Tables III and IV are directed to salts in standard intravenous solutions.

TABLE II

Solubility of Lyophilized and Crystalline Solids

| Combining Acid | Conc.[a] of CPD A mg/ml | | pH of Solution | | Conc.[a] of CPD B mg/ml | | pH of Solution | |
|---|---|---|---|---|---|---|---|---|
| | Lyo. | Cryst. | Lyo. | Cryst. | Lyo. | Cryst. | Lyo. | Cryst. |
| D-Galacturonic | 256 | 183 | 4.0 | 4.9 | | | | |
| Lactic | 256 | 183 | 4.6 | 5.15 | | | | |
| L-Aspartic | 256 | 183 | 4.5 | 4.8 | 246 | 183 | 4.65 | 4.80 |
| L-Glutamic | 254 | 183 | 5.2 | 5.5 | 246 | 183 | 4.8 | 4.84 |
| D-Gluconic[b] | 255 | 246 | 5.1 | 4.83 | 256 | 246 | 4.8 | 4.78 |
| Isethionic | | | | | 256 | 183 | 1.4 | 2.84 |
| Methane Sulphonic | | | | | 200 | 180 | 1.8 | 3.15 |
| Hydrochloric | | | | | | 23.2 | | 3 |

[a] Concentrations are not necessarily maximum values except for the hydrochloride.

[b] Gluconic acid δ-lactone used to prepare salts.

TABLE III

Effect of 10x Dilution of Soluble Forms of Compound A with Standard IV Solutions

| Combining Acid | Concen. of Cpd A mg/ml | pH of Solution | Solution 1 | | | Solution 2 | | | Solution 3 | | | Solution 4 | | |
|---|---|---|---|---|---|---|---|---|---|---|---|---|---|---|
| | | | pH dil | ppt 4 hr | ppt 24 hr | pH dil | ppt 4 hr | ppt 24 hr | pH dil | ppt 4 hr | ppt 24 hr | pH dil | ppt 4 hr | ppt 24 hr |
| L-Aspartic | 174 | 4.88 | 5.05 | | no | 4.83 | | slight | 4.84 | | no | 5.02 | | no |
| L-Glutamic | 174 | 5.08 | 5.18 | | no | 5.05 | | no | 5.05 | | no | 5.15 | | no |
| L-Cysteic | 166 | 2.92 | 3.17 | | no | 2.82 | | no | 2.68 | | no | 3.70 | | slight |
| Isethionic | 175 | 1.35 | 2.18 | | no | 2.27 | | no | 2.08 | | no | 3.70 | | no |
| D-Galacturonic | 166 | 5.44 | 5.36 | | no | 5.18 | | slight | 5.20 | | no | 5.36 | | no |
| D-Gluconic[a] | 182 | 4.30 | 4.4 | | no | 4.3 | | no | 4.4 | | no | 4.7 | | no |
| Lactic | 193 | 4.17 | 4.3 | | no | 4.8 | | no | 4.2 | | no | 4.5 | | no |
| Acetic | 235 | 5.3 | 5.4 | | no | 5.0 | | no | 5.3 | | no | 5.3 | | no |
| Hydrochloric | 201 | 1.52 | 2.7 | | no | 2.8 | | no | 2.7 | | no | 4.6 | | no |
| Methane Sulphonic | 173 | 1.5 | 5.4 | | no | 2.7 | | no | 2.5 | | no | 4.5 | | no |

Solution 1 0.9% NaCl

Solution 2 5% Dextrose

Solution 3 5% Dextrose + 0.9% NaCl

Solution 4 Lactated Ringer's Solution

[a] Commercially available gluconic acid solution utilized to prepare salt

## TABLE IV

Effect of 10x Dilution of Soluble Forms of Compound B with Standard IV Solution

| Combining Acid | Concen. of Cpd B mg/ml | pH of Solution | Solution 1 | | | Solution 2 | | |
|---|---|---|---|---|---|---|---|---|
| | | | pH dil | ppt 4 hr | ppt 24 hr | pH dil | ppt 4 hr | ppt 24 hr |
| L-Aspartic | 49.8 | 4.48 | 5.54 | | no | | | |
| | 165 | 4.64 | 4.82 | no | yes | | | |
| | 190 | 4.60 | | | | 4.83 | | no |
| L-Glutamic | 48.2 | 4.95 | 4.99 | | no | | | |
| | 160 | 4.98 | 5.10 | no | yes | | | |
| | 154 | 5.20 | | | | 5.06 | slight | slight |
| L-Cysteic | 52 | 2.96 | 3.29 | | no | | | |
| | 221 | 2.78 | 2.8 | yes | yes | 2.9 | hazy | hazy |
| Isethionic | 48.8 | 2.16 | 3.05 | | no | | | |
| | 220 | 1.01 | 2.08 | no | yes | | | |
| | 196 | 1.40 | | | | 2.35 | | no |
| D-Galacturonic | 45.4 | 4.42 | 4.47 | | no | | | |
| | 155 | 4.78 | 4.8 | | no | 4.8 | | no |
| D-Gluconic[a] | 168 | 4.6 | 4.6 | no | yes | 4.5 | | no |
| Lactic | 221 | 4.04 | 4.1 | yes | yes | 4.2 | hazy | hazy |
| Acetic | 195 | 4.97 | 5.2 | yes | yes | 5.6 | slight | slight |
| Methane Sulphonic | 26.2 | 5.07 | 4.47 | | no | | | |
| | 178 | 1.38 | 2.3 | yes | yes | 3.2 | hazy | hazy |

Solution 1: 0.9% NaCl Solution 2: 5% Dextrose

[a] Commercially available gluconic acid solution utilized to prepare salt.

TABLE IV (Continued)

| Combining Acid | Concen. of CPD B mg/ml | pH of Solution | Solution 3 | | | Solution 4 | | |
|---|---|---|---|---|---|---|---|---|
| | | | pH dil | ppt 4 hr | ppt 24 hr | pH dil | ppt 4 hr | ppt 24 hr |
| L-Aspartic | 49.8 | 4.48 | | | | | | |
| | 165 | 4.64 | | | | | | |
| | 190 | 4.60 | 4.71 | | yes | 4.94 | | no |
| L-Glutamic | 48.2 | 4.95 | | | | | | |
| | 160 | 4.98 | | | | | | |
| | 154 | 5.20 | 4.95 | | slight | 5.09 | | slight |
| L-Cysteic | 52 | 2.96 | | | | | | |
| | 221 | 2.78 | 2.8 | yes | yes | 3.9 | yes | yes |
| Isethionic | 48.8 | 2.16 | | | | | | |
| | 220 | 1.01 | | | | | | |
| | 196 | 1.40 | 2.14 | | yes | 3.95 | | slight |
| D-Galacturonic | 45.4 | 4.42 | | | | | | |
| | 155 | 4.78 | 4.8 | | no | 5.1 | | no |
| D-Gluconic[a] | 168 | 4.6 | 4.8 | no | yes | 4.6 | | no |
| Lactic | 221 | 4.04 | 4.1 | yes | yes | 4.3 | yes | yes |
| Acetic | 195 | 4.97 | 5.4 | yes | yes | 5.3 | slight | yes |
| Methane Sulphonic | 26.2 | 5.07 | | | | | | |
| | 178 | 1.38 | 2.8 | yes | yes | 4.2 | yes | yes |

Solution 3: 5% Dextrose + 0.9% NaCl    Solution 4: Lactated Ringer's Solution

[a] Commercially available gluconic acid solution utilized to prepare salt

The compounds of the present invention are useful in treating diseases originating with organisms of the genera *Straphylococcus, Streptococcus, Haemophilus, Neisseria, Clostridium, Enterobacter, Escherichia, Klebsiella, Proteus, Providencia, Pseudomonas* and *Serratia*.

While the salts of the present invention may be administered orally, they are preferably administered parenterally, with the dosage adjusted to the needs and tolerances of the individual patient. Of the numerous parenteral routes, the intravenous route is most preferred. The usual mammalian dosage range for a 70 kg human subject is from 70 mg to 21 g per day (1 mg to 300 mg per kg of weight per day), preferably from 210 mg to 6.3 g per day (3 mg to 90 mg per kg of weight per day), optionally in divided portions.

The following Tables V to X demonstrate that the compounds of the present invention have both in vitro and in vivo antibacterial activity when tested by standard procedures.

TABLE V

Antibacterial Activity[a] Comparison of Compound A and its Salts

| Compound | MIC $\mu g/ml$ | | | | | | | | | |
|---|---|---|---|---|---|---|---|---|---|---|
| | Entero cloacae MA 2646 | E. coli Vogel | Klebs. pneumoniae MGH–2 | Proteus retgeri M 1771 | Pseudo. aeruginosa UI–18 | Staph. aureus H 228 | Staph. aureus UC–76 | Strep. faecalis MGH–2 | Strep. pneumoniae SV–1 | Strep. pyogenes C–203 |
| A | <0.1 | 0.1 | 0.2 | <0.1 | 0.4 | 1.6 | 0.1 | 1.6 | 0.8 | 0.8 |
| Aspartate | 0.1 | <0.1 | 0.2 | <0.1 | <0.1 | 0.8 | <0.1 | 1.6 | 1.6 | 0.8 |
| Glutamate | <0.1 | <0.1 | 0.1 | 0.1 | 0.1 | 1.6 | <0.1 | 1.6 | 1.6 | 0.8 |
| Gluconate | 0.2 | <0.1 | 0.2 | <0.1 | 0.2 | 1.6 | 0.2 | 0.8 | 1.6 | 0.8 |
| Galacturonate | 0.1 | <0.1 | 0.1 | <0.1 | 0.1 | 0.8 | <0.1 | 1.6 | 1.6 | 0.8 |

[a] Based on content of compound A.

TABLE VI

Antibacterial Activity[a] Comparison of Compound B and its Salts

| Compound | MIC $\mu g/ml$ | | | | | | | | | |
|---|---|---|---|---|---|---|---|---|---|---|
| | Entero cloacae MA 2646 | E. coli Vogel | Klebs. pneumoniae MGH-2 | Proteus retgeri M 1771 | Pseudo. aeruginosa UI-18 | Staph. aureus H 228 | Staph. aureus UC-76 | Strep. faecalis MGH-2 | Strep. pneumoniae SV-1 | Strep. pyogenes C-203 |
| B | 0.2 | 0.2 | 0.1 | 0.1 | 0.8 | 3.1 | 0.2 | 1.6 | 3.1 | 3.1 |
| Aspartate | 0.2 | <0.1 | 0.1 | <0.1 | 0.4 | 3.1 | 0.1 | 3.1 | 3.1 | 6.3 |
| Glutamate | 0.2 | <0.1 | 0.2 | 0.1 | 0.2 | 1.6 | <0.1 | 3.1 | 3.1 | 6.3 |
| Gluconate | 0.4 | 0.1 | 0.2 | 0.2 | 0.8 | 1.6 | 0.4 | 3.1 | 3.1 | 3.1 |

[a] Based on content of compound B.

TABLE VII

Antibacterial Activity Comparison of Compound A with its Gluconate Salt by Subcutaneous (SC) Route in Acute Mouse Protection Test[a]

| Compound | Single sc Dose $PD_{50}$ (mg/kg) ± 95% Confidence Limits | |
|---|---|---|
| | Klebsiella pneumoniae MGH–2 | Escherichia coli Vogel |
| A | 1.3 ± 0.3 | 0.5 ± 0.1 |
| Gluconate | 1.4 ± 0.3 | 0.5 ± 0.1 |

[a] Single dose of drug given at time of challenge.

TABLE VIII

Antibacterial Activity Comparison of Compound A with its Gluconate Salt by Oral (PO) Route in Acute Mouse Protection Test[a]

| Compound | Single po Dose $PD_{50}$ (mg/kg) ± 95% Confidence Limits | |
|---|---|---|
| | Klebsiella pneumoniae MGH–2 | Escherichia coli Vogel |
| A | 15 ± 3 | 3.3 ± 0.5 |
| Gluconate | 13 ± 6 | 3.9 ± 0.9 |

[a] Single dose of drug given at time of challenge.

TABLE IX

Antibacterial Activity Comparison of Compound B and Salts by Subcutaneous (SC) Route in Acute Mouse Protection Test[a]

| Compound | Single sc Dose $PD_{50}$ (mg/kg) ± 95% Confidence Limits | | |
|---|---|---|---|
| | Staphylococcus aureus UC–76 | Escherichia coli Vogel | Klebsiella pneumoniae MGH–2 |
| B | 7.4 ± 2.2 | 1.4 ± 0.4 | 2.6 ± 0.6 |
| Aspartate | 4.3 ± 1.4 | 1.3 ± 0.4 | 1.6 ± 0.6 |
| Glutamate | 2.8 ± 1.0 | 1.6 ± 0.6 | 1.9 ± 0.9 |
| Gluconate | --- ± --- | 1.1 ± 0.4 | 5.2 ± 1.5 |

[a] Single dose of drug given at time of challenge.

TABLE X

Antibacterial Activity Comparison of Compound B and Salts by Oral (PO) Route in Acute Mouse Protection Test[a]

| Compound | Single po Dose $PD_{50}$ (mg/kg) ± 95% Confidence Limits | |
|---|---|---|
| | Staphylococcus aureus UC–76 | Escherichia coli Vogel |
| B | 14 ± 7.0 | 3.1 ± 0.7 |
| Aspartate | 13 ± 3.0 | 2.9 ± 1.0 |
| Glutamate | 12.0 ± 3.0 | 2.9 ± 1.0 |
| Gluconate | --- ± --- | 2.9 ± 0.7 |

[a] Single dose of drug given at time of challenge.

The usefulness of the salts of the present invention for oral administration was further demonstrated by the following experiment:

In a two-way crossover trial, four Beagle dogs were given single 25 mg/kg (parent compound equivalent) oral doses of compound B and compound D as the glutamate in hard gelatine capsules. Drug blood levels were determined by a disk agar diffusion microbiological assay.

Based on statistical analysis of the blood level data there was no significant difference in peak blood level or drug half-life between compound B and compound D as the glutamate. This indicates the usefulness of the salts of the invention for oral administration.

The pharmaceutical compositions of the invention may be produced by formulating a compound of formula C (as the active ingredient) in dosage unit form with a pharmaceutical carrier. Some examples of nonparenteral dosage unit forms are tablets, capsules, lozenges, and pills; as well as powders, aqueous and non-aqueous oral solutions and suspensions and suppositories. Preferably parenteral solutions are used which are packaged in containers containing either one or some larger number of dosage units.

For purposes of the present invention a parenteral dosage form may also comprise a premeasured portion of a dry compound of structural formula C which is intended to be reconstituted to a solution prior to use. The dry material may be prepared by lyophilization of a solution or may be a recrystallized material. This type dosage form may optionally contain additional materials such as buffers, neutral salts, sugars, acids and the like. In practice, the parenteral dosage form comprising the lyophilized or crystalline material is dissolved in a pharmaceutically acceptable liquid such as water which may optionally contain buffers, preservatives, materials to make the resultant solution isotonic or other materials known to those skilled in the art. All such materials should be compatible with the compounds of the present invention. The reconstituted solution so prepared may be used for direct parenteral injection or may be added to a solution such as an I.V. solution for slow administration by infusion.

Some examples of suitable pharmaceutical carriers for oral administration, including pharmaceutical diluents, are sugars such as lactose and sucrose; starches such as corn starch and potato starch; cellulose derivatives such as sodium carboxymethyl cellulose, ethyl cellulose, methyl cellulose, and cellulose acetate phthalate; gelatin, talc; stearic acid; magnesium stearate; propylene glycol; glycerine and water. Oral compositions of the invention can also contain other components such as thickeners, sweeteners, colouring and flavouring agents. These materials, if present, are usually employed in relatively small amounts.

Injectable carriers include glucose, isotonic saline, and buffered solutions; as well as other compatible substances normally used in parenteral pharmaceutical formulations.

Suppositories would employ glycerine, cocoa butter, and the like as vehicles. The suppositories are generally prepared by melting the desired vehicle, adding the active component, thoroughly mixing, pouring the molten mixture into suitable moulds, and allowing the thus formed shape dosage forms to cool.

The compositions can, if desired, also contain other therapeutic agents. The percentage of the active ingredient in the foregoing compositions can be varied within wide limits but for practical purposes is preferably present in a concentration of at least 10% in a solid composition and at least 2% in a primarily

liquid composition. The most satisfactory compositions are those in which a much higher proportion of the active ingredient is present. The compositions of the invention preferably contain from 50 mg to 5 mg of the active ingredient per dosage unit so that the entire amount of active desired to be administered during a day can be made up from a reasonable number of dosage units.

The present invention is illustrated by the following examples.

Example 1

1-Ethyl-6-fluoro-1,4-dihydro-4-oxo-7-(1-piperazinyl)-1,8-naphthyridine-3-carboxylic acid L-aspartate.

A mixture of 199 mg (1.5 mmoles) of L-aspartic acid, 480 mg (1.5 mmoles) of 1-ethyl-6-fluoro-1,4-dihydro-4-oxo-7-(1-piperazinyl)-1,8-naphthyridine-3-carboxylic acid and 10 ml of water was warmed to 60°C. The abbreviation "mmole" represents millimole. The resulting solution was freeze dried to give 730 mg of solid. A 600 mg portion of the solid was crystallized from a solution of 2 ml of water and 4 ml of absolute ethanol. The crystals were filtered, washed with absolute ethanol and dried to give 310 mg of product; mp 214—215°C dec., $[\alpha]_D^{23}$ −4.3° (c 2, $H_2O$). Analysis showed the sample contained 0.8 mole of water per mole of compound.

Example 2

1-Ethyl-6-fluoro-1,4-dihydro-4-oxo-7-(1-piperazinyl)-1,8-naphthyridine-3-carboxylic acid L-glutamate.

A mixture of 220 mg (1.5 moles) of L-glutamic acid, 480 mg (1.5 mmoles) of 1-ethyl-6-fluoro-1,4-dihydro-4-oxo-7-(1-piperazinyl)-1,8-naphthyridine-3-carboxylic acid and 10 ml of water was warmed to 60°. The resulting solution was freeze dried to give 690 mg of solid. A 620 mg portion of the solid was crystallized from a solution of 2 ml of water and 6 ml of absolute ethanol. The crystals were filtered, washed with absolute ethanol and dried to give 484 mg of product; mp 196—200° dec., $[\alpha]_D^{23}$ −0.9 (c 2, $H_2O$). Analysis showed the sample contained 0.6 mole of water per mole of compound.

Example 3

1-Ethyl-6-fluoro-1,4-dihydro-4-oxo-7-(1-piperazinyl)-3-quinolinecarboxylic acid L-aspartate.

A mixture of 1.28 g (4 mmoles) of 1-ethyl-6-fluoro-1,4-dihydro-4-oxo-7-(1-piperazinyl)-3-quinolinecarboxylic acid, 532 mg (4 mmoles) of L-aspartic acid and 30 ml of water was stirred at 40—50° for 20 min and at room temperature for 2.5 hr. The solution was clarified by filtration and the filtrate was freeze dried to give 1.77 g of solid. The material was crystallized from 6 ml of water and 20 ml of absolute ethanol. The crystals were filtered, washed with absolute ethanol and diethyl ether, and dried to give 1.11 g of product; mp 208° dec., $[\alpha]_D^{23}$ −5.1° (c 2, $H_2O$). Analysis showed that the sample contained 0.8 mole of water per mole of compound.

Example 4

1-Ethyl-6-fluoro-1,4-dihydro-4-oxo-7-(1-piperazinyl)-3-quinolinecarboxylic acid L-glutamate.

A mixture of 1.28 g (4 mmoles) of 1-ethyl-6-fluoro-1,4-dihydro-4-oxo-7-(1-piperazinyl)-3-quinolinecarboxylic acid, 588 mg (4 mmoles) of L-glutamic acid, and 50 ml of water was warmed on the steam bath. The resulting solution was clarified by filtration and the filtrate was lyophilized to give 1.81 g of solid. The material was crystallized from 6 ml of water and 45 ml of absolute ethanol. The crystals were filtered, washed with absolute ethanol and diethyl ether and dried to give 1.39 g of product; mp 184—185° dec; $[\alpha]_D^{23}$ +1.3° (c 2, $H_2O$). Analysis showed the sample contained 0.1 mole of ethanol and 0.25 mole of water per mole of compound.

Example 5

1-Ethyl-6-fluoro-1,4-dihydro-4-oxo-7-(1-piperazinyl)-3-quinolinecarboxylic acid D-galacturonate.

A mixture of 1.28 g (4 mmoles) of 1-ethyl-6-fluoro-1,4-dihydro-4-oxo-7-(1-piperazinyl)-3-quinolinecarboxylic acid, 817 mg (4 mmoles) of D-galacturonic acid hydrate and 30 ml of water was stirred for 1.5 hours at room temperature. The solution was clarified by filtration and the filtrate was freeze dried to give 1.98 of solid. The material was crystallized from a solution of 7.5 ml of water and 14 ml of absolute ethanol. The crystals were filtered, washed with absolute ethanol and diethyl ether and dried to give 1.36 g of product; mp 142° dec., $[\alpha]_D^{23}$ +8.4° (c 2, $H_2O$). Analysis showed the sample contained 0.2 mole of ethanol and 0.15 mole of water per mole of compound.

Example 6

1-Ethyl-6-fluoro-1,4-dihydro-4-oxo-7-(1-piperazinyl)-1,8-naphthyridine-3-carboxylic acid D-gluconate

Method A

A suspension of 1.39 g (4 mmoles) of 1-ethyl-6-fluoro-1,4-dihydro-4-oxo-7-(1-piperazinyl)-1,8-naphthyridine-3-carboxylic acid sesquihydrate, 712 mg (4 mmoles) of D-gluconic acid δ-lactone and 30 ml of water was stirred at room temperature for two hours. The suspension was briefly warmed to 55—60°Cand stirring was continued at room temperature for one hour. Insolubles were removed by filtration and the filtrate was freeze dried. The solid was crystallized from a solution of 4 ml of water and 24 ml of absolute ethanol. The crystals were filtered, washed with absolute ethanol and diethyl ether and dried to give 1.08 g of product; mp 165—167°C dec., $[\alpha]_D^{23}$ +4.8°(c 2, $H_2O$). Analysis showed the sample contained 0.7 mole of water per mole of compound.

Method B

A suspension of 2.78 g (8 mmoles) of 1-ethyl-6-fluoro-1,4-dihydro-4-oxo-7-(1-piperazinyl)-1,8-naphthyridine-3-carboxylic acid sesquihydrate, 1.21 ml (2.8 mmoles) of 2.34 N commercial D-gluconic acid solution, and 60 ml of water was stirred at room temperature. After 12 hours an additional 2.7 ml (6.3 mmoles) of commercial D-gluconic acid solution in water was added and yellow solution resulted within five minutes. The solution was clarified by filtration and the filtrate freeze dried. The solid was crystallized from a solution of about 10 ml of water and 40 ml of absolute ethanol. The crystals were stirred for four hours at room temperature, filtered, washed with absolute ethanol and diethyl ether and dried to give 3.97 g of product; mp 164—165° dec., $[\alpha]_D^{23}$ +4.9° (c 2, $H_2O$). Analysis showed that the sample contained about 1 mole of water per mole of compound.

Example 7

1-Ethyl-6-fluoro-1,4-dihydro-4-oxo-7-(1-piperazinyl)-3-quinolinecarboxylic acid D-gluconate

Method A

A suspension of 2.7 g (8 mmoles) of 1-ethyl-6-fluoro-1,4-dihydro-4-oxo-7-(1-piperazinyl)-3-quinolinecarboxylic acid monohydrate, 1.43 g (8 mmoles) of D-gluconic acid δ-lactone and 60 ml of water was stirred at room temperature for 19.5 hours. The solution was clarified by filtration and the filtrate was freeze dried. The solid was crystallized from a solution of about 10 ml of water and 50 ml of absolute ethanol. The crystals were filtered, washed with absolute ethanol and diethyl ether and dried to give 3.56 g of product; mp 171—172°C dec., $[\alpha]_D^{23}$ +5.1° (c 2, $H_2O$). Analysis showed the sample contained 0.1 mole of water per mole of compound and was slightly hygroscopic.

Method B

A suspension of 0.98 g (2.9 mmoles) of 1-ethyl-6-fluoro-1,4-dihydro-4-oxo-7-(1-piperazinyl)-3-quinolinecarboxylic acid monohydrate, 2.48 ml (2.9 mmoles) of 1.17N D-gluconic acid in water from dilution of a commercial solution and 20 ml of water was stirred at room temperature for two hours. The yellow solution was clarified by filtration and the filtrate was freeze dried. The solid was crystallized from a solution of about 4 ml of water and 17 ml of absolute ethanol. The crystals were filtered, washed with absolute ethanol and diethyl ether and dried to give 1.35 g of product as crystals; mp 165—168°C dec., $[\alpha]_D^{23}$ +5.7 (c 2, $H_2O$). Analysis showed the sample contained about 0.2 mole of water per mole of compound.

The D-gluconate salts prepared from the commercial gluconic acid solution (Examples 6B and 7B) were observed to give lower decomposition points than the corresponding salts prepared using D-gluconic acid delta lactone. The gluconate salts prepared from the commercial D-glyconic acid solution were also more highly coloured (yielding darker solutions) than the corresponding salts prepared using D-gluconic acid delta lactone. Gluconate salt formation using D-gluconic acid delta lactone is therefore the preferred technique for the process of the present invention.

Example 8

Lyophilized Injectable Formulation

Compound B as the sesquihydrate (22.6 g) and D-gluconic acid δ lactone (23.2 g) were added to 250 ml water for injection, briefly heated to 55°C and stirred for three hours while cooling to room temperature. The resulting solution was filtered thorugh a 0.2 μ filter, and 4.8—5.2 ml aliquots of solution were introduced into sterile vials and lyophilized. Yield: approximately 50 vials each containing about 645 mg compound D as the gluconate and about 245 mg excess gluconic acid.

Compound B as the sesquihydrate (22.6 g) and D-gluconic acid δ lactone (12.2 g) were added to 250 ml water for injection, briefly heated to 55° and stirred for three hours while cooling to room temperature. The resulting solution was filtered through a 0.2 μ filter, and 4.8—5.2 ml aliquots of solution were introduced into sterile vials and lyophilized. Yield: approximately 50 vials each containing about 654 mg compound D as the gluconate.

Compound A as the monohydrate (21.9 g) and D-gluconic acid δ lactone (23.2 g) were added to 250 ml water for injection, heated briefly to 55°C and stirred for three hours while returning to room temperature. The resulting solution was filtered thorugh a 0.2 μ filter, and 4.8—5.2 ml aliquots of solution were introduced into sterile vials and lyophilized. Yield: approximately 50 vials each containing about 645 mg compound E as the gluconate and about 245 mg excess gluconic acid.

Compound A as the monohydrate (21.9 g) and D-gluconic acid δ lactone (23.2 g) were added to 250 ml water for injection, heated briefly to 55°C and stirred for about three hours while returning to room temperature. The resulting solution was filtered through 0.2 μ filter, and 4.8—5.3 ml aliquots of solution were introduced into sterile vials and lyophilized. Yield: approximately 50 vials each containing about 645 mg compound E as the gluconate.

Example 9

Solution for Injection

A solution was prepared from compound B sesquihydrate 22.6 g and D-gluconic acid δ lactone 23.2 g in 250 ml water for injection as described in Example 8. Approximately 5 ml aliquots of the resulting solution were introduced respectively into 50 sterile ampoules and sealed.

A solution was prepared from compound A monohydrate (21.9 g) and D-gluconic acid δ lactone (23.2 g) in 250 ml water for injection as described in Example 8. Approximately 5 ml aliquots of the resulting solution were introduced respectively into 50 sterile ampoules and sealed.

Example 10

Capsules

Compound D as the gluconate
100 mg, 250 mg, or 500 mg

| | |
|---|---|
| Compound D as the gluconate | 500 g |
| Lactose USP, Anhydrous q.s. or | 200 g |
| Sterotex Powder HM | 5 g |

Compound D as the gluconate and the lactose were combined in a twin-shell blender equipped with an intensifier bar. The mixture was tumble blended for two minutes, followed by blending for one minute with the intensifier bar and then tumble blended again for one minute. A portion of the blend was then mixed with the sterotex powder, passed through a No. 30 screen, and added back to the remainder of the blend. The mixed ingredients were then blended for one minute, blended with the intensifier bar for thirty seconds, and tumble blended for an additional minute. Appropriate sized capsules were filled with 141 mg, 352.5 mg, or 705 mg of the blend, respectively, for the 100 mg, 250 mg, and 500 mg containing capsules.

Compound E as the gluconate
100 mg, 250 mg, or 500 mg

| | |
|---|---|
| Compound E as the gluconate | 500 g |
| Lactose USP, Anhydrous q.s. or | 200 g |
| Sterotex Powder HM | 5 g |

The mixing and filling was carried out as described above to produce 100 mg, 250 mg, and 500 mg containing capsules.

Example 11

Tablets

Compound D as the gluconate
100 mg, 250 mg, or 500 mg

| | |
|---|---|
| Compound D as the gluconate | 500 g |
| Corn Starch NF | 200.0 g |
| Cellulose, Microcrystalline | 46.0 g |
| Sterotex Powder HM | 4.0 g |
| Purified Water q.s. or | 300.0 ml |

The corn starch, the cellulose, and compound D as the gluconate were combined together in a planetary mixer and mixed for two minutes. Water was added to this combination and mixed for one minute. The resulting mix was spread on trays and dried in a hot air oven at 50°C until a moisture level of 1 to 2 percent was obtained. The dried mix was then milled with a Fitzmill through a No. RH2B screen at medium speed. The sterotex powder was added back to the milled mixture and the total blended for five minutes by drum rolling. Compressed tablets of 150 mg, 375 mg, and 750 mg, respectively, of the total mix are formed with appropriate sized punches for the 100 mg, 250 mg, or 500 mg containing tablets.

In a similar manner, tablets are prepared from compound E as the gluconate.

Example 12

Suppositories

Compound D as the gluconate
250 mg, 500 mg, or 1000 mg per 3 g suppository

| | | | |
|---|---|---|---|
| Compound D as the gluconate | 250 mg | 500 mg | 1000 mg |
| Polyethylene Glycol 1540 | 1925 mg | 1750 mg | 1400 mg |
| Polyethylene Glycol 8000 | 825 mg | 750 mg | 600 mg |

The polyethylene glycol 1540 and the polyethylene glycol 8000 were melted together at 60°C, and compound D as the gluconate was then dissolved into the melt. The resulting product was then moulded at 25°C into appropriate suppositories.

The term "q.s. or" appearing in certain of the foregoing Examples indicates that either a sufficient quantity or the indicated amount may be used, the choice being left to the practitioner.

**Claims for the Contracting States: BE CH DE FR GB IT LI LU NL SE**

1. A compound having the following general formula C:

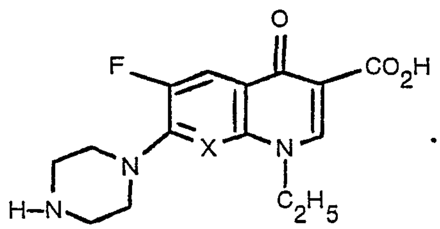

·Z (C)

wherein X is N or CH and Z is galacturonic, aspartic, gluconic or glutamic acid.

2. A compound having the following general formula D:

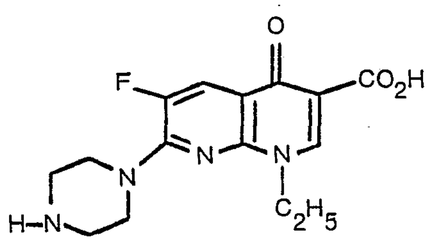

·Z (D)

wherein Z is galacturonic, aspartic, gluconic or glutamic acid.

3. A compound having the following general formula E:

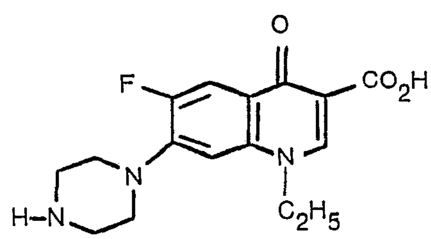

·Z (E)

wherein Z is galacturonic, aspartic, gluconic or glutamic acid.

4. The compound claimed in Claim 1, which is 1-ethyl-6-fluoro-1,4-dihydro-4-oxo-7-(1-piperazinyl)-1,8-naphthyridine-3-carboxylic acid galacturonate.

5. The compound claimed in Claim 1, which is 1-ethyl-6-fluoro-1,4-dihydro-4-oxo-7-(1-piperazinyl)-1,8-naphthyridine-3-carboxylic acid aspartate.

6. The compound defined in Claim 1 which is 1-ethyl-6-fluoro-1,4-dihydro-4-oxo-7-(1-piperazinyl)-1,8-naphthyridine-3-carboxylic acid gluconate.

7. The compound claimed in Claim 1, which is 1-ethyl-6-fluoro-1,4-dihydro-4-oxo-7-(1-piperazinyl)-1,8-naphthyridine-3-carboxylic acid glutamate.

8. The compound claimed in Claim 1, which is 1-ethyl-6-fluoro-1,4-dihydro-4-oxo-7-(1-piperazinyl)-3-quinolinecarboxylic acid galacturonate.

9. The compound claimed in Claim 1, which is 1-ethyl-6-fluoro-1,4-dihydro-4-oxo-7-(1-piperazinyl)-3-quinolinecarboxylic acid aspartate.

10. The compound claimed in Claim 1, which is 1-ethyl-6-fluoro-1,4-dihydro-4-oxo-7-(1-piperazinyl)-3-quinolinecarboxylic acid gluconate.

11. The compound claimed in Claim 1, which is 1-ethyl-6-fluoro-1,4-dihydro-4-oxo-7-(1-piperazinyl)-3-quinolinecarboxylic acid glutamate.

12. An antibacterial pharmaceutical composition comprising a compound as claimed in any preceding claim, in combination with a pharmaceutically acceptable carrier.

13. A process for preparing a compound having the following general formula C;

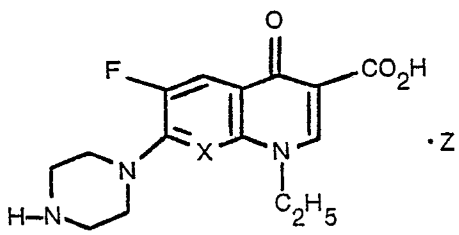

(C)

wherein X is N or CH and Z is galacturonic, aspartic, gluconic or glutamic acid; which process comprises reacting a compound having the following general formula:

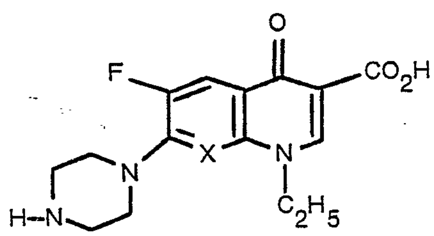

wherein X is defined above, with an acid of formula Z as defined above.

**Claims for the Contracting State: AT**

1. A process for preparing a compound having the following general formula C:

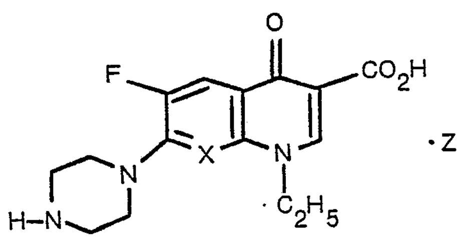

(C)

wherein X is N or CH and Z is galacturonic, aspartic, gluconic or glutamic acid; which process comprises reacting a compound having the following general formula (F):

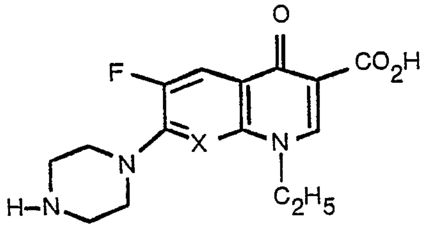

(F)

wherein X is defined above, with an acid of formula Z as defined above.

2. A process for producing a compound having the following general formula D:

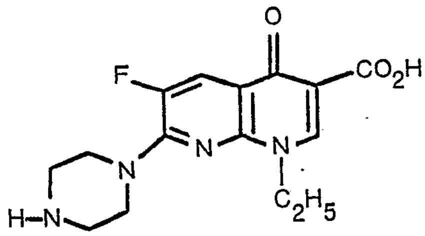

· Z

(D)

wherein Z is galacturonic, aspartic, gluconic or glutamic acid, which process comprises reacting a compound having the following formula (B):

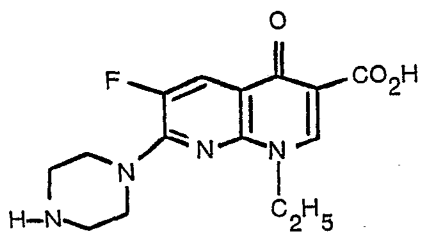

(B)

with an acid of formula Z as defined above.

3. A process for producing a compound having the following general formula E:

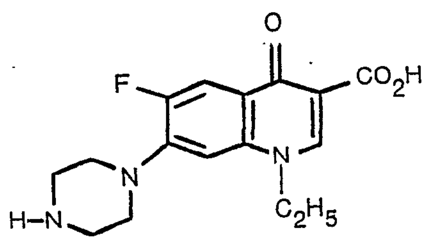

· Z

(E)

wherein Z is galacturonic, aspartic, gluconic or glutamic acid, which process comprises reacting a compound having the following formula (A):

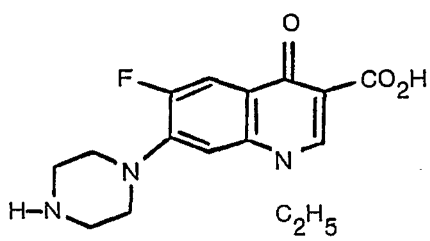

(A)

with an acid of formula Z as defined above.

4. A process according to Claim 2, for producing 1-ethyl-6-fluoro-1,4-dihydro-4-oxo-7-(1-piperazinyl)-1,8-naphthyridine-3-carboxylic acid galacturonate;
1-ethyl-6-fluoro-1,4-dihydro-4-oxo-7-(1-piperazinyl)-1,8-naphthyridine-3-carboxylic acid aspartate; or
1-ethyl-6-fluoro-1,4-dihydro-4-oxo-7-(1-piperazinyl)-1,8-naphthyridine-3-carboxylic acid glutamate, wherein Z is galacturonic, aspartic or glutamic acid, respectively.

5. A process according to Claim 2, for producing 1-ethyl-6-fluoro-1,4-dihydro-4-oxo-7-(1-piperazinyl)-1,8-naphthyridine-3-carboxylic acid gluconate, wherein Z is gluconic acid.

6. A process according to Claim 3, for producing 1-ethyl-6-fluoro-1,4-dihydro-4-oxo-7-(1-piperazinyl)-3-quinolinecarboxylic acid galacturonate;
1-ethyl-6-fluoro-1,4-dihydro-4-oxo-7-(1-piperazinyl)-3-quinolinecarboxylic acid aspartate; or
1-ethyl-6-fluoro-1,4-dihydro-4-oxo-7-(1-piperazinyl)-3-quinolinecarboxylic acid glutamate, wherein Z is galacturonic, aspartic or glutamic acid, respectively.

7. A process according to Claim 3, for producing 1-ethyl-6-fluoro-1,4-dihydro-4-oxo-7-(1-piperazinyl)-3-quinolinecarboxylic acid gluconate, wherein Z is gluconic acid.

8. A process for producing an antibacterial pharmaceutical composition which comprises combining a compound of formula C as defined in Claim 1, with a pharmaceutically acceptable carrier.

**Patentansprüche für die Vertragsstaaten: BE CH DE FR GB IT LI LU NL SE**

1. Verbindung mit der folgenden allgemeinen Formel (C)

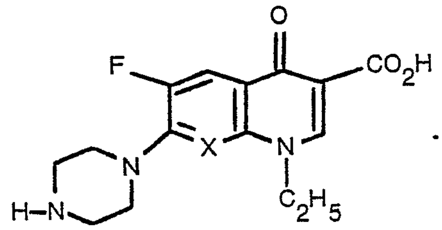 · Z (C)

worin X für N oder CH steht und Z Galacturonsäure, Asparaginsäure, Glukonsäure oder Glutaminsäure ist.

2. Verbindungen der folgenden allgemeinen Formel (D)

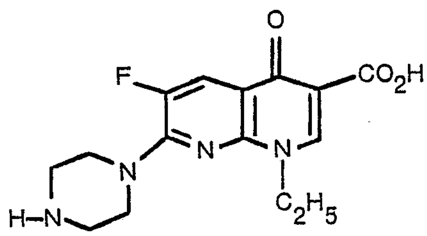 · Z (D)

worin Z Galacturonsäure, Asparaginsäure, Glukonsäure oder Gluatminsäure repräsentiert.

3. Verbindung der folgenden allgemeinen Formel (E)

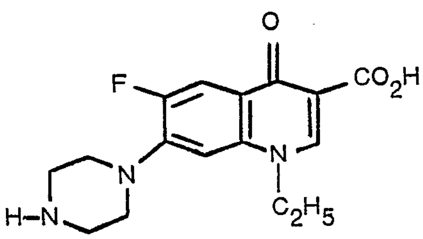 · Z (E)

worin Z für Galacturonsäure, Asparaginsäure, Glukonsäure oder Glutaminsäure steht.

4. Verbinung nach Anspruch 1, welche 1-Äthyl-6-fluor-1,4-dihydro-4-oxo-7-(1-piperazinyl)-1,8-naphthyridin-3-carbonsäuregalacturonat bedeutet.

5. Verbindung nach Anspruch 1, welche 1-Äthyl-6-fluor-1,4-dihydro-4-oxo-7-(1-piperazinyl)-1,8-naphthyridin-3-carbonsäureasparaginat ist.

6. Verbindung nach Anspruch 1, welche 1-Äthyl-6-fluor-1,4-dihydro-4-oxo-7-(1-piperazinyl)-1,8-naphthyridin-3-carbonsäuregluconat ist.

7. Verbindung nach Anspruch 1, welche 1-Äthyl-6-fluor-1,4-dihydro-4-oxo-7-(1-piperazinyl)-1,8-naphthyridin-3-carbonsäureglutamat ist.

8. Verbindung nach Anspruch 1, welche 1-Äthyl-6-fluor-1,4-dihydro-4-oxo-7-(1-piperazinyl)-3-chinolincarbonsäuregalacturonat ist.

9. Verbindung nach Anspruch 1, welche 1-Äthyl-6-fluor-1,4-dihydro-4-oxo-7-(1-piperazinyl)-3-chinolincarbonsäureasparaginat ist.

10. Verbindung nach Anspruch 1, welche 1-Äthyl-6-fluor-1,4-dihydro-4-oxo-7-(1-piperazinyl)-3-chinolincarbonsäuregluconat ist.

11. Verbindung nach Anspruch 1, welche 1-Äthyl-6-fluor-1,4-dihydro-4-oxo-7-(1-piperazinyl)-3-chinolincarbonsäureglutamat ist.

12. Antibakterielle pharmazeutische Zusammensetzung, dadurch gekennzeichnet, daß eine Verbindung nach irgendeinem der vorhergehenden Ansprüche mit einem pharmazeutisch akzeptablen Träger kombiniert ist.

13. Verfahren zur Herstellung einer Verbindung der folgenden allgemeinen Formel (C)

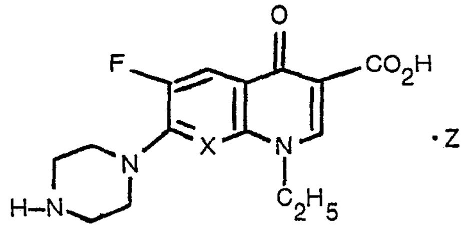

(C)

worin X für N oder CH steht, und Z Galaturonsäure, Asparaginsäure, Glukonsäure oder Glutaminsäure ist; welches Verfahren dadurch gekennzeichnet ist, daß eine Verbindung der folgenden allgemeinen Formel

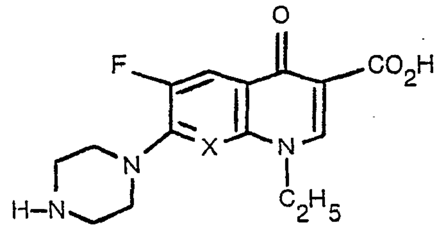

worin X wie oben definiert ist, mit einer Säure der wie oben definierten Formel (Z) umgesetzt wird.

**Patentansprüche für den Vertragsstaat: AT**

1. Verfahren zur Herstellung einer Verbindung der allgemeinen Formel (C)

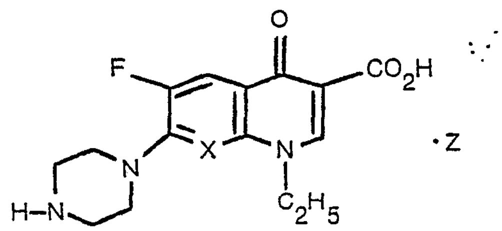

(C)

worin X gleich N oder CH ist, und Z Galacturonsäure, Asparaginsäure, Glukonsäure oder Glutaminsäure repräsentiert; welches Verfahren dadurch gekennzeichnet ist, daß eine Verbindung der folgenden allgemeinen Formel (F)

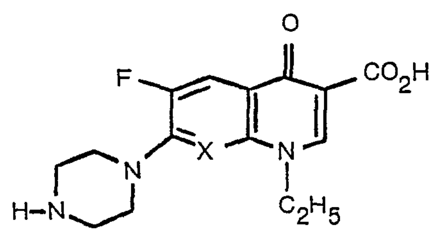

(F)

worin X wie oben definiert ist, mit einer Säure der wie oben definierten Formel Z umgesetzt wird.

2. Verfahren zur Herstellung einer Verbindung der folgenden allgemeinen Formel (D)

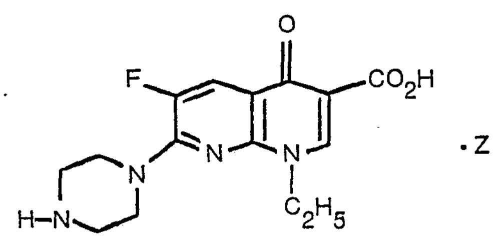

(D)

worin Z Galacturonsäure, Asparaginsäure, Glukonsäure oder Glutaminsäure ist, welches Verfahren

dadurch gekennzeichnet ist, daß eine Verbindung der allgemeinen Formel (B)

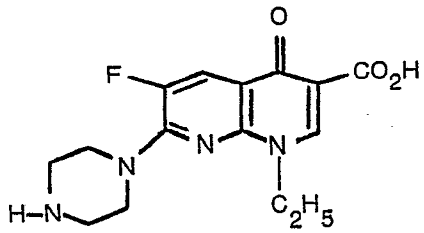

(B)

mit einer Säure der wie oben definierten Formel Z umgesetzt wird.

3. Verfahren zur Herstellung einer Verbindung der allgemeinen Formel (E)

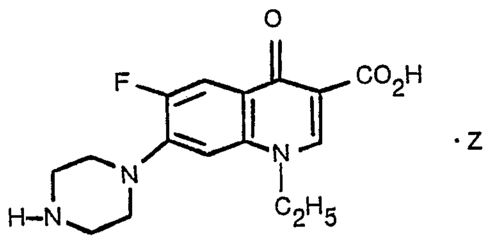

(E)

worin Z Galacturonsäure, Asparaginsäure, Glukonsäure oder Glutaminsäure ist, welches Verfahren dadurch gekennzeichnet ist, daß eine Verbindung mit der folgenden Formel (A)

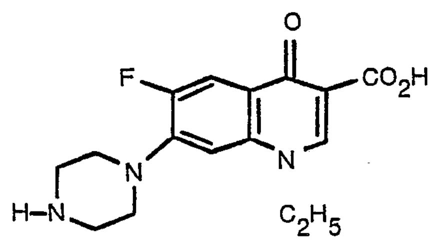

(A)

mit einer Säure der wie oben definierten Fomrel Z umgesetzt wird.

4. Verfahren nach Anspruch 2 zur Herstellung von 1-Äthyl-6-fluor-1,4-dihydro-4-oxo-7-(1-piperazinyl)-1,8-naphthyridin-3-carbonsäuregalacturonat; 1-Äthyl-6-fluor-1,4-dihydro-4-oxo-7-(1-piperazinyl)-1,8-naphthyridin-3-carbonsäureasparaginat; oder 1-Äthyl-6-fluor-1,4-dihydro-4-oxo-7-(1-piperazinyl)-1,8-naphthyridin-3-carbonsäureglutamat, worin Z jeweils Galacturonsäure, Asparaginsäure oder Glutaminsäure ist.

5. Verfahren nach Anspruch 2 zur Herstellung von 1-Äthyl-6-fluor-1,4-dihydro-4-oxo-7-(1-piperazinyl)-1,8-naphthyridin-3-carbonsäureglukonat, worin Z Glukonsäure ist.

6. Verfahren nach Anspruch 3 zur Herstellung von 1-Äthyl-6-fluor-1,4-dihydro-4-oxo-7-(1-piperazinyl)-3-chinolincarbonsäuregalacturonat; 1-Äthyl-6-fluor-1,4-dihydro-4-oxo-7-(1-piperazinyl)-3-chinolincarbonsäureasparaginat; oder 1-Äthyl-6-fluor-1,4-dihydro-4-oxo-7-(1-piperazinyl)-3-chinolincarbonsäure glutamat, worin Z jeweils Galacturonsäure, Asparginsäure oder Glutaminsäure ist.

7. Verfahren nach Anspruch 3 zur Herstellung von 1-Äthyl-6-fluor-1,4-dihydro-4-oxo-7-(1-piperazinyl)-3-chinolincarbonsäureglukonat, worin Z Glukonsäure ist.

8. Verfahren zur Herstellung einer antibakteriellen pharmazeutischen Zusammensetzung, dadurch gekennzeichnet, daß eine Verbindung der in Anspruch 1 definierten Formel C mit einem pharmazeutisch akzeptablen Träger kombiniert ist.

**Revendications pour les Etats contractants: BE CH DE FR GB LI LU NL SE IT**

1. Un composé de formule générale C suivante:

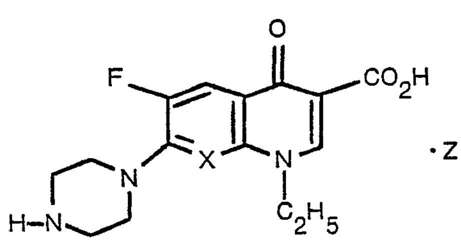

(C)

dans laquelle:

X représente N ou CH; et

Z représente l'acide galacturonique, aspartique, gluconique ou glutamique.

2. Un composé de formule générale D suivante:

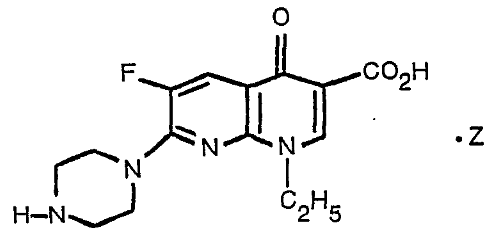

(D)

dans laquelle:

Z représente l'acide galacturonique, aspartique, gluconique ou glutamique.

3. Un composé de formule générale E suivante:

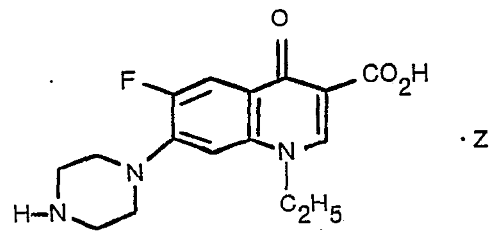

(E)

dans laquelle:

Z représente l'acide galacturonique, aspartique, gluconique ou glutamique.

4. Le composé selon la revendication 1, caractérisé en ce qu'il s'agit du galacturonate de l'acide 1-éthyl-6-fluoro-1,4-dihydro-4-oxo-7-(1-pipérazinyl)-1,8-naphtyridine-3-carboxylique.

5. Le composé selon la revendication 1, caractérisé en ce qu'il s'agit de l'aspartate de l'acide 1-éthyl-6-fluoro-1,4-dihydro-4-oxo-7-(1-pipérazinyl)-1,8-naphtyridine-3-carboxylique.

6. Le composé selon la revendication 1, caractérisé en ce qu'il s'agit du gluconate de l'acide 1-éthyl-6-fluoro-1,4-dihydro-4-oxo-7-(1-pipérazinyl)-1,8-naphtyridine-3-carboxylique.

7. Le composé selon la revendication 1, caractérisé en ce qu'il s'agit du glutamate de l'acide 1-éthyl-6-fluoro-1,4-dihydro-4-oxo-7-(1-pipérazinyl)-1,8-naphtyridine-3-carboxylique.

8. Le composé selon la revendication 1, caractérisé en ce qu'il s'agit du galacturonate de l'acide 1-éthyl-6-fluoro-1,4-dihydro-4-oxo-7-(1-pipérazinyl)-3-quinolinecarboxylique.

9. Le composé selon la revendication 1, caractérisé en ce qu'il s'agit de l'asparate de l'acide 1-éthyl-6-fluoro-1,4-dihydro-4-oxo-7-(1-pipérazinyl)-3-quinolinecarboxylique.

10. Le composé selon la revendication 1, caractérisé en ce qu'il s'agit du gluconate de l'acide 1-éthyl-6-fluoro-1,4-dihydro-4-oxo-7-(1-pipérazinyl)-3-quinolinecarboxylique.

11. Le composé selon la revendication 1, caractérisé en ce qu'il s'agit du glutamate de l'acide 1-éthyl-6-fluoro-1,4-dihydro-4-oxo-7-(1-pipérazinyl)-3-quinolinecarboxylique.

12. Composition pharmaceutique antibactérienne comprenant un composé selon l'une quelconque des revendications précédentes, en combinaison avec un véhicule pharmaceutiquement acceptable.

13. Procédé de préparation d'un composé de formule générale suivante C:

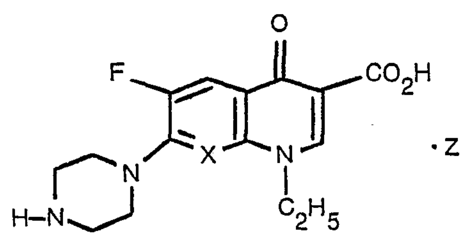

(C)

dans laquelle:

X représente N ou CH; et

Z représente l'acide galacturonique, aspartique, gluconique ou glutamique, ce procédé consistant à faire réagir un composé de formule générale suivante:

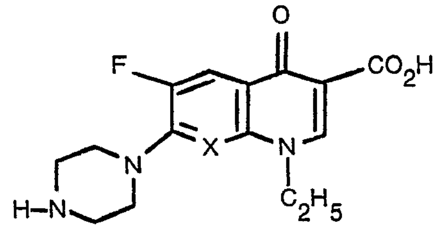

dans laquelle:

X est tel que défini ci-dessus, avec un acide de formule Z tel que défini ci-dessus.

**Revendications pour l'Etat contractant: AT**

1. Procédé de préparation d'un composé de formule générale C suivante:

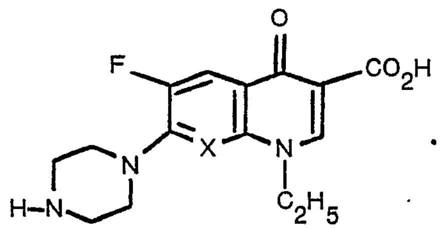

•Z (C)

dans laquelle:

X représente N ou CH; et

Z représente l'acide galacturonique, aspartique, gluconique ou glutamique,

ce procédé consistant à faire réagir un composé de formule générale suivante (F):

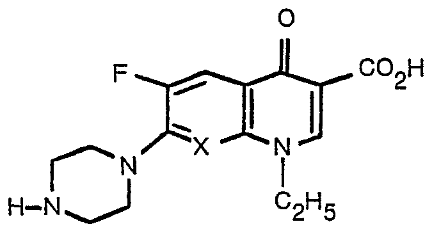

(F)

dans laquelle:

X tel que défini ci-dessus, avec un acide de formule Z tel que défini ci-dessus.

2. Procédé de préparation d'un composé de formule générale D suivante:

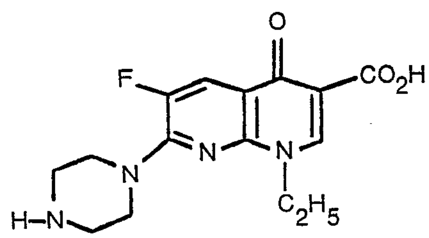

•Z (D)

dans laquelle:

Z représente l'acide galacturonique, aspartique, gluconique ou glutamique, ce procédé consistant à faire réagir un composé de formule suivante (B):

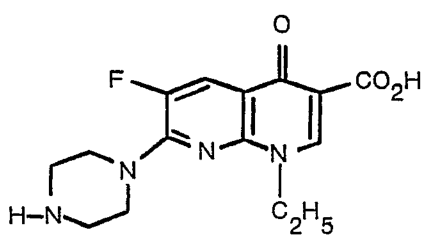

(B)

avec un acide de formule Z tel que défini ci-dessus.

3. Procédé de préparation d'un composé de formule générale E suivante:

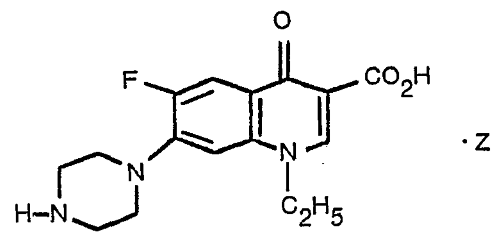

(E)

dans laquelle:

Z représente l'acide galacturonique, aspartique, gluconique ou glutamique, ce procédé consistant à faire réagir un composé de formule suivante (A):

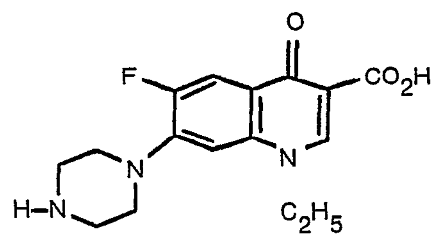

(A)

avec un acide de formule Z tel que défini ci-dessus.

4. Procédé selon la revendication 2 de préparation du galacturonate de l'acide 1-éthyl-6-fluoro-1,4-dihydro-4-oxo-7-(1-pipérazinyl)-1,8-naphtyridine-3-carboxylique; de l'aspartate de l'acide 1-éthyl-6-fluoro-1,4-dihydro-4-oxo-7-(1-pipérazinyl)-1,8-naphtyridine-3-carboxylique ou du glutamate de l'acide 1-éthyl-6-fluoro-1,4-dihydro-4-oxo-7-(1-pipérazinyl)-1,8-naphtyridine-3-carboxylique, dans lequel Z représente respectivement l'acide galacturonique, l'acide aspartique ou l'acide glutamique.

5. Procédé selon la revendication 2, de préparation du gluconate de l'acide 1-éthyl-6-fluoro-1,4-dihydro-4-oxo-7-(1-pipérazinyl)-1,8-naphtyridine-3-carboxylique, dans lequel Z représente l'acide gluconique.

6. Procédé selon la revendication 3 de préparation du galacturonate de l'acide 1-éthyl-6-fluoro-1,4-dihydro-4-oxo-7-(1-pipérazinyl)-3-quinolinecarboxylique; de l'aspartate de l'acide 1-éthyl-6-fluoro-1,4-dihydro-4-oxo-7-(1-pipérazinyl)-3-quinolinecarboxylique; du glutamate de l'acide 1-éthyl-6-fluoro-1,4-dihydro-4-oxo-7-(1-pipérazinyl)-3-quinolinecarboxylique, dans lequel Z représente respectivement l'acide galacturonique, l'acide aspartique ou l'acide glutamique.

7. Procédé selon la revendication 3, de préparation du gluconate de l'acide 1-éthyl-6-fluoro-1,4-dihydro-4-oxo-7-(1-pipérazinyl)-3-quinolinecarboxylique, dans lequel Z représente l'acide gluconique.

8. Procédé de préparation d'une composition pharmaceutique antibactérienne combiné à un composé de formule C tel que défini dans la revendication 1 à un véhicule pharmaceutique acceptable.